# EUROPÄISCHE PATENTSCHRIFT

(11) **EP 1 358 861 B1**
(45) Veröffentlichungstag und Bekanntmachung des Hinweises auf die Patenterteilung: **11.03.2009**
(21) Anmeldenummer: 03009186.2
(22) Anmeldetag: 22.04.2003
(51) Int. Cl.: A61F 2/44, A61F 2/30

(54) **Wirbelsäulenimplantat**
Spinal implant
Implant vertébral

(30) Priorität: 23.04.2002 DE 10218093
(43) Veröffentlichungstag der Anmeldung: 05.11.2003
(73) Patentinhaber: Signus Medizintechnik GmbH, 63755 Alzenau (DE)
(72) Erfinder: Siedler, Uwe, 63755 Alzenau (DE)
(74) Vertreter: Leinweber & Zimmermann

(56) Entgegenhaltungen:
- EP-A- 0 517 030
- EP-A- 0 904 751
- WO-A-00/45751
- WO-A-01/70136
- WO-A-01/70139
- US-A- 5 192 327
- US-A1- 2002 029 084

## Beschreibung

Die Erfindung bezieht sich auf ein Wirbelsäulenimplantat mit zwei Implantatelementen, die in eingesetztem Zustand jeweils eine dem benachbarten Wirbel zugewandte Verankerungsseite besitzen, welcher jeweils eine Sicherheit gegen Verschieben in allen Richtungen gewährleistende Verzahnung zugeordnet ist, und die in ihrer relativen Position in bezug zueinander fixierbar sind.

Bei einem bekannten Wirbelsäulenimplantat dieser Art (DE 199 02 481 A) sind zwei das Implantat bildende Implantatelemente vorgesehen, die in bezug zueinander axial verschiebbar geführt und in der gewünschten Relativposition gegeneinander verspannbar sind. Zu diesem Zweck besteht das eine der beiden Elemente aus sich achsparallel erstreckenden Segmenten, die mit Hilfe einer sich quer zu Erstreckungsrichtung wirksamen Spannschraube in radialer Richtung biegbar und dadurch gegeneinander verspannbar sind.

Diese Ausführung ist aufwendig in der Herstellung und bei ihrem Einsatz schwierig in der Handhabung.

Ausweislich des Oberbegriffs von Anspruch 1 geht die Erfindung von einem Wirbelsäulenimplantat aus, wie es aus der EP-A-0 517 030 bekannt ist.

Der Erfindung liegt die Aufgabe zugrunde, das bekannte Wirbelsäulenimplantat in baulicher Hinsicht zu verbessern.

Erfindungsgemäß wird die gestellte Aufgabe mit einem Wirbelsäulenimplantat nach Anspruch 1 gelöst.

Auf diese Weise läßt sich ein den jeweiligen Gegebenheiten entsprechend bemessenes Wirbelsäulenimplantat schaffen, das stabil und auch bei größerer Implantathöhe hoch belastbar ist. Die Höhe des Wirbelsäulenimplantats hängt dabei von der Zahl der übereinander angeordneten Implantatelemente, jedoch auch von der jeweiligen Stärke der einzelnen Elemente ab.

Als besonders zweckmäßig im Hinblick auf eine sichere Verbindung der einzelnen Implantatelemente hat sich die Tatsache erwiesen, daß die der gegenseitigen Fixierung aufeinanderfolgender Implantatelemente dienende Steckverbindung von den Implantatelementen achsparallel vorstehende Zungen mit jeweils einer sich quer erstreckenden Haltekante umfaßt, durch die eine Stützkante des benachbarten Implantatelements erfassbar ist.

Zweckmäßigerweise besitzen die Implantatelemente mindestens eine Bohrung, durch die hindurch die Stützkante von der Haltekante der Zunge des benachbarten Implantatelements erfassbar ist. So ist gewährleistet, daß sich die Zungen gegen äußere Einwirkungen geschützt im Inneren des Stapels aufeinanderfolgender Implantatelemente befinden. Außerdem ist in vorteilhafter Weise die Möglichkeit gegeben, die von der Haltekante der Zunge des benachbarten Implantatelements erfassbare Stützkante durch den Rand der vorzugsweise zentralen Bohrung des benachbarten Implantatelements zu bilden, was den baulichen Aufwand des Wirbelsäulenimplantats vermindert.

Dabei ergibt sich eine besonders einfache Ausführung, wenn vier Implantatelemente vorgesehen sind, nämlich die beiden äußeren Elemente, die jeweils eine dem benachbarten Wirbel zugewandte Verankerungsseite aufweisen, das durch ein zentrales Grundelement gebildete, zwischenliegende Implantatelement mit den beiden Stützkanten und mindestens einem als Verbindungselement dienenden Implantatelement, das die Verbindung zwischen jeweils dem einen der beiden äußeren Implantatelemente und dem zentralen Grundelement herstellt.

Weiterhin ist in äußerst zweckmäßiger Weise vorgesehen, daß den Implantatelementen, die mit Stützkanten für das Erfassen der Haltekanten der eingeführten Zungen versehen sind, zu den axialen Bohrungen führende Querbohrungen zugeordnet sind, durch die hindurch die die Rastverbindung aufeinanderfolgender Implantatelemente sicherstellenden Zungen im Sinne einer seitlichen elastischen Verformung zur Lösung der Rastverbindung beaufschlagbar sind. Zu diesem Zweck findet eine Zange mit auf die Lage und Größe der Querbohrungen abgestimmten Vorsprüngen Anwendung, mit deren Hilfe durch die Querbohrungen hindurch die Zungen quer beaufschlagbar sind, um die Rastverbindung im Bedarfsfall auf sehr einfache Weise zu lösen.

In weiterer sehr zweckmäßiger Ausbildung des Wirbelsäulenimplantats ist vorgesehen, daß die Implantatelemente in Form rechteckiger Quader ausgebildet und auf ihrer in Richtung Grundelement dem angrenzenden Implantatelement zugewandten Seite an einer ihrer Kanten mit einer Ausnehmung versehen sind, in die ein am angrenzenden Implantatelement vorgesehener Vorsprung eingreift. Diese Ausgestaltung stellt sicher, daß jeweils zueinander passende Quaderseiten aneinander zur Anlage gelangen, um das fertige Wirbelsäulenimplantat zu bilden. In besonders günstiger Weise ist die Ausnehmung durch eine Abschrägung gebildet, die mit einem am angrenzenden Implantetelement vorgesehenen Vorsprung in Form einer prismatischen Leiste zusammenwirkt.

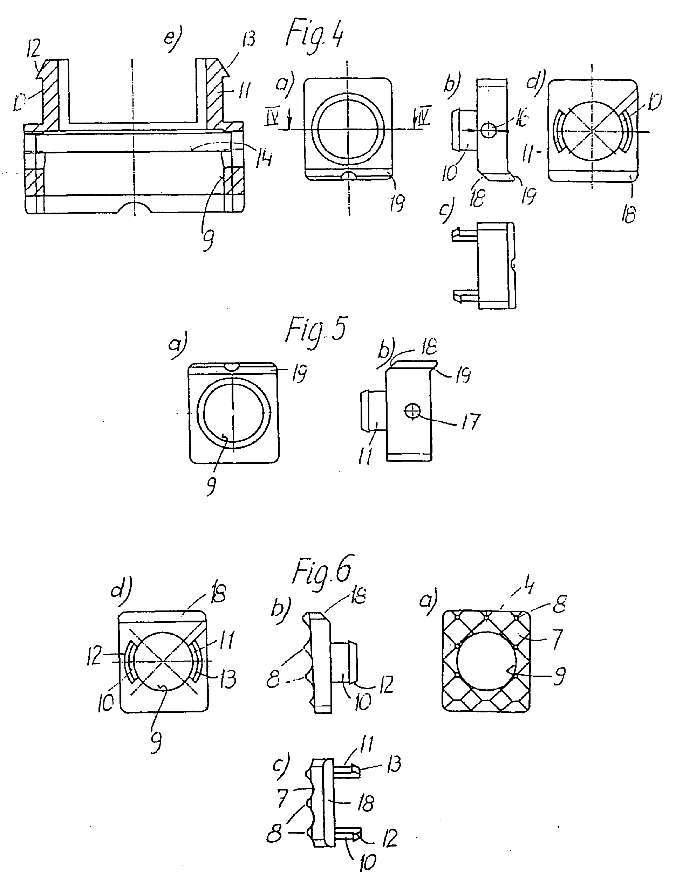

Weitere zweckmäßige Einzelheiten und Vorteile des erfindungsgemäßen Wirbelsäulenimplantats ergeben sich aus der folgenden Beschreibung eines Ausführungsbeispiels anhand der beigefügten Zeichnung im Maßstab 2:1, und zwar zeigen:
- Fig. 1: eine perspektivische Ansicht eines aus fünf Elementen bestehenden Wirbelsäulenimplantats, in Explosions-Darstellung,
- Fig. 2: a) ein erstes äußeres Implantatelement in einer Stirnansicht,
b) einer Seitenansicht,
c) einer Draufsicht,
d) einer Rückansicht und
e) einer Schnittansicht entsprechend der Linie II-II der Fig. 2a),
- Fig. 3: ein zentrales Grundelement in
a) einer Stirnansicht,
b) einer Seitenansicht,
c) einer Draufsicht,
d) einer Rückansicht,
e) einer Schnittansicht entsprechend der Linie III-III der Fig. 3a),
f) einer Schnittansicht eines Details aus Fig. 3e) in größerem Maßstab 5:1.
- Fig.: 4 ein erstes zwischenliegendes Implantatelement in
a) einer Stirnansicht,
b) einer Seitenansicht,
c) einer Draufsicht,
d) einer Rückansicht,
e) einer Schnittansicht entsprechend der Linie IV-IV der Fig. 4a) in größerem Maßstab 5:1
- Fig. 5: ein zweites zwischenliegendes Implantatelement in
a) einer Stirnansicht,
b) einer Seitenansicht, und
- Fig. 6: eine zweites äußeres Implantatelement in
a) einer Stirnansicht,
b) einer Seitenansicht,
c) einer Draufsicht und
d) einer Rückansicht.

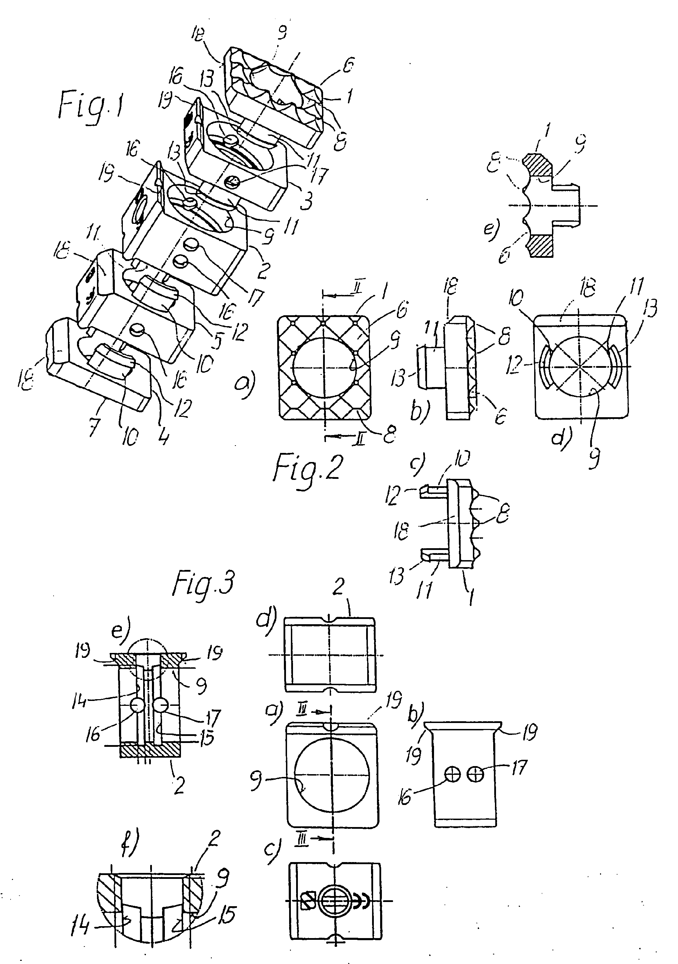

Wie aus Fig. 1 ersichtlich, besteht das veranschaulichte Wirbelsäulenimplantat gemäß einer bevorzugten Ausführungsform aus fünf Implantatelementen, nämlich einem ersten äußeren Implantatelement 1, einem zentralen Grundelement 2, einem zwischen dem ersten äußeren Implantatelement 1 und dem zentralen Grundelement 2 liegenden Implantatelement 3, einem zweiten äußeren Implantatelement 4 und einem zwischen dem zentralen Grundelement 2 und dem zweiten äußeren Implantatelement 4 liegenden Implantatelement 5. Diese fünf Implantatelemente sind miteinander fest verbindbar und bilden im verbundenen Zustand ein Wirbelsäulenimplantat nach der Erfindung. Die beiden äußeren Implantatelemente 3 und 4 besitzen jeweils eine dem benachbarten Wirbel zugewandte Verankerungsseite 6 und 7 mit Ausnehmungen, die, wie in den Fig. 2 und 6 verdeutlicht, in Spitzen 8 auslaufende Flanken besitzen, die miteinander einen Winkel von ca. 90° einschließen. Diese Spitzen 8 bilden eine Sicherheit gegen Verschieben des Wirbelsäulenimplantats in allen Richtungen gewährleistende Verzahnung. Auf ihrer der Verankerungsseite 6 bzw. 7 abgelegenen Seite sind die beiden äußeren Implantatelemente 3 und 4 jeweils mit sich achsparallel längs der Wandung einer zentralen Bohrung 9 erstreckenden Zungen 10, 11 versehen. Diese weisen an ihren Enden außenseitig sich quer erstreckende Haltekanten 12 bzw. 13 auf, die in zusammengestecktem Zustand jeweils eine Stützkante im Bereich der Bohrung des angrenzenden Implantatelements hintergreifen, d.h. erfassen. Diese Zungen 10, 11 bilden somit eine Steckverbindung, über die die Implantatelemente 1 bis 5 zu einem in sich stabilen Wirbelsäulenimplantat miteinander verbindbar sind.

Anstelle einer einzigen zentralen Bohrung 9 können natürlich auch zwei exzentrische Bohrungen vorgesehen sein, in die jeweils eine der Zungen einführbar ist.

Wie aus der Zeichnung, insbesondere Fig. 1 ersichtlich, sind die beiden äußeren Implantatelemente 3 und 4 jeweils über die Steckverbindungszungen 10, 11 mit dem jeweils folgenden Implantatelement verbunden. Dieses steht seinerseits über analoge Zungen mit einem durch ein zwischenliegendes zungenloses Grundelement 2 gebildeten Implantatelement in Verbindung, das - wie Fig. 3f) in größerem Maßstab 5 : 1 zeigt - in seiner mittleren Zone zwei Stützkanten 14, 15 für jeweils eine der beiden in die Bohrungen 9 eingeführten Haltekanten 12, 13 der Zungen 10, 11 aufweist.

Sowohl Fig. 1 als auch Fig. 3b) und e) zeigen deutlich, daß im Bereich der Haltekanten 12 bzw. 13 der in Einrastposition befindlichen Zungen 10 bzw. 11 zu den axialen Bohrungen 9 führende Querbohrungen 16, 17 vorgesehen sind, durch die hindurch die die Rastverbindung aufeinanderfolgender Implantatelemente sicherstellenden Zungen im Sinne einer seitlichen elastischen Verformung zur Lösung der Rastverbindung beaufschlagbar sind. Dazu dient zweckmäßigerweise eine nicht näher veranschaulichte Zange mit zwei aufeinanderzu gerichteten Fingern. Der Zeichnung ist ferner entnehmbar, daß die Implantatelemente 1 bis 6 jeweils in Form rechteckiger Quader ausgebildet sind. Auf ihrer in Richtung dem Grundelement 2 zugewandten Seite sind die Implantatelemente 1 und 3 bis 5 an einer ihrer Kanten mit einer Ausnehmung in Form einer Abschrägung 18 versehen, an der ein am angrenzenden Implantatelement vorgesehener Vorsprung 19 in Form einer prismatischen Leiste zur Anlage gelangt. Das Zusammenspiel von Abschrägung 18 und Vorsprung 19 stellt sicher, daß die Implantatelemente automatisch ihre optimale Position zueinander einnehmen.

Während Fig. 2 das eine äußere Verankerungsseite 6 aufweisende Implantatelement 1 mit gleichmäßiger Stärke zeigt, ist das in Fig. 6 veranschaulichte, ebenfalls eine äußere Verankerungsseite 7 aufweisende Implantatelement 4 ungleichmäßig stark ausgebildet. Es weist vielmehr eine von der einen Außenseite zur anderen Außenseite, an der sich die Abschrägung 18 befindet, leicht zunehmende Stärke auf, so daß das zu einer Einheit zusammengesetzte Wirbelsäulenimplantat keine parallel zueinander verlaufenden Verankerungsseiten 6, 7 besitzt.

Den Abmessungsverhältnissen der Zeichnung ist bei Berücksichtigung der Darstellungsmaßstäbe entnehmbar, daß sich aus den Implantatelementen mit einer Stärke im Bereich von 3 bis 8 mm ein Wirbelsäulenimplantat unterschiedlicher Höhe in einem Bereich von vorzugsweise 20 bis knapp 50 mm zusammensetzen läßt. Natürlich müssen dabei nicht unbedingt fünf Elemente zu einem Implantat zusammengefügt werden. Das modulare System läßt es vielmehr zu, daß auf das äußere Implantatelement 1 unmittelbar das zwischenliegende Grundelement 2 folgt, an das sich ggf. über das zweite zwischenliegende Implantatelement 5 das zweite äußere Implantatelement 4 anschließt.

## Patentansprüche

1. Wirbelsäulenimplantat mit zwei äußeren Implantatelementen (1, 4), die in eingesetztem Zustand jeweils eine dem benachbarten Wirbel zugewandte Verankerungsseite (6, 7) besitzen, welcher jeweils eine Sicherheit gegen Verschieben in allen Richtungen gewährleistende Verzahnung (8) zugeordnet ist, und die in ihrer relativen Position in bezug zueinander fixierbar sind, wobei mehrere Implantatelemente (2, 3, 5) zusammen mit den äußeren Implantatelementen (1, 4) nach Art eines modularen Systems zu der gewünschten Implantathöhe stapelbar und mittels einer Steckverbindung (10 bis 14) in bezug zueinander fixierbar sind,
**dadurch gekennzeichnet, daß**
die beiden äußeren Implantatelemente (1, 4) über Steckverbindungszungen (10, 11) mit dem jeweils folgenden Implantatelement verbunden sind und daß ein dazwischenliegendes Grundelement (2) vorhanden ist, das lediglich Stützkanten (14, 15) für das Erfassen von Haltekanten (12, 13) der von beiden Seiten her eingeführten Zungen (10, 11) der angrenzenden Implantatelemente aufweist.

2. Wirbelsäulenimplantat nach Anspruch 1, **dadurch gekennzeichnet, daß** die der gegenseitigen Fixierung aufeinanderfolgender Implantatelemente (1 bis 5) dienende Steckverbindung von den Implantatelementen (1, 3, 4, 5) achsparallel vorstehende Zungen (10, 11) mit jeweils einer sich quer erstreckenden Haltekante (12, 13) umfaßt, durch die eine Stützkante (14) des benachbarten Implantatelements (2, 3, 5) erfaßbar ist.

3. Wirbelsäulenimplantat nach Anspruch 2, **dadurch gekennzeichnet, daß** die Implantatelemente mindestens eine Bohrung (9) besitzen, durch die hindurch die Stützkante (14) von der Haltekante (12, 13) der Zunge (10, 11) des benachbarten Implantatelements (1, 3, 4, 5) erfaßbar ist.

4. Wirbelsäulenimplantat nach Anspruch 3, **dadurch gekennzeichnet, daß** die von der Haltekante (12, 13) der Zunge (10, 11) des benachbarten Implantatelements (1, 3, 4, 5) erfaßbare Stützkante (14) durch einen Rand der Bohrung (9) des benachbarten Implantatelements (2) gebildet ist.

5. Wirbelsäulenimplantat nach einem der Ansprüche 1 bis 4, **dadurch gekennzeichnet, daß** vier Implantatelemente vorgesehen sind, nämlich die beiden äußeren Elemente (1, 4), die jeweils eine dem benachbarten Wirbel zugewandte Verankerungsseite (6, 7) aufweisen, das durch ein zentrales Grundelement gebildete, zwischenliegende lmplantatelemente mit den beiden Stützkanten (14, 15) und mindestens einem als Verbindungselement dienenden Implantatelement (3, 5), das die Verbindung zwischen jeweils dem einen der beiden äußeren lmplantatelemente (1, 4) und dem zentralen Grundelement (2) herstellt.

6. Wirbelsäulenimplantat nach einem der Ansprüche 2 bis 5, **dadurch gekennzeichnet, daß** den Implantatelementen (2, 3, 5), die mit Stützkanten für das Erfassen der Haltekanten (12, 13) der eingeführten Zungen (10, 11) versehen sind, zu den axialen Bohrungen (9) führende Querbohrungen (16, 17) zugeordnet sind, durch die hindurch die die Rastverbindung aufeinanderfolgender Implantatelemente sicherstellenden Zungen (10, 11) im Sinne einer seitlichen elastischen Verformung zur Lösung der Rastverbindung beaufschlagbar sind.

7. Wirbelsäulenimplantat nach einem der Ansprüche 1 bis 6, **dadurch gekennzeichnet, daß** die Implantatelemente (1 bis 5) in Form rechteckiger Quader ausgebildet und auf ihrer in Richtung Grundelement (2) dem angrenzenden Implantatelement zugewandten Seite an einer ihrer Kanten mit einer Ausnehmung (18) versehen sind, in die ein am angrenzenden Implantatelement vorgesehener Vorsprung (19) eingreift.

8. Wirbelsäulenimplantat nach Anspruch 7, **dadurch gekennzeichnet, daß** die Ausnehmung durch eine Abschrägung (18) gebildet wird, die mit einem am angrenzenden Implantatelement vorgesehenen Vorsprung (19) in Form einer prismatischen Leiste zusammenwirkt.

## Claims

1. A spinal column implant with two outer implant elements (1, 4) which in the fitted state respectively have an anchoring side (6, 7) facing towards the adjacent vertebra to which toothing (8) guaranteeing security against movement in all directions is respectively assigned, and which can be fixed in their relative position in relation to one another, a number of implant elements (2, 3, 5) together with the outer implant elements (1, 4) being stackable in the manner of a modular system to the desired implant height and being fixable in relation to one another by means of a plug-in connection (10 to 14),
**characterised in that that**
the two outer implant elements (1, 4) are connected by means of plug-in connection tongues (10, 11) to the respectively following implant element and that a base element (2) lying between the latter is provided which only has support edges (14, 15) for engaging holding edges (12, 13) of the tongues (10, 11) introduced from the sides of the adjacent implant elements.

2. The spinal column implant according to Claim 1, **characterised in that** the plug-in connection serving to fix consecutive implant elements (1 to 5) reciprocally comprises tongues projecting axis-parallel from the implant elements (1, 3, 4, 5) respectively with a holding edge (12, 13) extending laterally by means of which the one support edge (14) of the adjacent implant element (2, 3, 5) can engage.

3. The spinal column implant according to Claim 2, **characterised in that** the implant elements have at least one borehole (9) through which the support edge (14) of the holding edge (12, 13) of the tongue (10, 11) of the adjacent implant element (1, 3, 4, 5) can engage.

4. The spinal column implant according to Claim 3, **characterised in that** the support edge (14) which can be engaged by the holding edge (12, 13) of the tongue (10, 11) of the adjacent implant element (1, 3, 4, 5) is formed by an edge of the borehole (9) of the adjacent implant element (2).

5. The spinal column implant according to any of Claims 1 to 4, **characterised in that** four implant elements are provided, namely the two outer elements (1, 4) which respectively have an anchoring side (6, 7) facing towards the adjacent vertebra, the intermediate implant element formed by a central base element with the two support edges (14, 15) and at least one implant element (3, 5) serving as a connection element, which produces the connection between respectively one of the two outer implant elements (1, 4) and the central base element (2).

6. The spinal column implant according to any of Claims 2 to 5, **characterised in that** lateral boreholes (16, 17) leading to the axial boreholes (9) are assigned to the implant elements (2, 3, 5) which are provided with support edges for engagement of the holding edges (12, 13) of the inserted tongues (10, 11) through which the tongues (10, 11) securing the snap-on connection of consecutive implant elements can be stressed in the sense of an elastic deformation at the side in order to release the snap-on connection.

7. The spinal column implant according to any of Claims 1 to 6, **characterised in that** the implant elements (1 to 5) are designed in the form of rectangular cuboids and are provided on their side facing towards the adjacent implant element in the direction of the base element (2) on one of their edges with a recess (18) in which a projection (19) provided on the adjacent implant element (19) engages.

8. The spinal column implant according to Claim 7, **characterised in that** the recess is formed by a slant (18) which interacts with a projection (19) in the form of a prismatic bar provided on the adjacent implant element.

## Revendications

1. Implant vertébral comportant deux éléments d'implant extérieurs (1, 4) qui possèdent respectivement, à l'état inséré, un côté d'ancrage (6, 7) orienté vers la vertèbre adjacente et auquel est associé une denture (8) assurant une sécurité contre tout déplacement dans une quelconque direction, et qui sont susceptibles d'être fixés dans leur position relative l'un par rapport à l'autre, plusieurs éléments d'implant (2, 3, 5) pouvant être empilés avec les éléments d'implant extérieurs (1, 4) à la manière d'un système modulaire pour fournir la hauteur de l'implant désirée et pouvant être fixés les uns par rapport aux autres au moyen d'une connexion enfichable (10 à 14),
**caractérisé en ce que**
les deux éléments d'implant extérieurs (1, 4) sont raccordés à l'élément d'implant respectivement consécutif grâce à des pattes de connexion enfichables (10, 11) et **en ce qu'**il est prévu un élément principal (2) intercalé qui présente uniquement des bords d'appui (14, 15) destinés à saisir les bords de retenue (12, 13) des pattes (10, 11) des éléments d'implant contigus introduites des deux côtés.

2. Implant vertébral selon la revendication 1, **caractérisé en ce que** la connexion enfichable qui sert à la fixation mutuelle des éléments d'implant (1 à 5) consécutifs comprend des pattes (10, 11) faisant saillie de manière axialement parallèle à partir des éléments d'implant (1, 3, 4, 5) et présentant respectivement un bord de retenue (12, 13) à prolongement transversal grâce auquel un bord d'appui (14) de l'élément d'implant (2, 3, 5) adjacent est susceptible d'être saisi.

3. Implant vertébral selon la revendication 2, **caractérisé en ce que** les éléments d'implant possèdent au moins une perforation (9) à travers laquelle le bord d'appui (14) est susceptible d'être saisi par le bord de retenue (12, 13) de la patte (10, 11) de l'élément d'implant (1, 3, 4, 5) adjacent.

4. Implant vertébral selon la revendication 3, **caractérisé en ce que** le bord d'appui (14) saisi par le bord de retenue (12, 13) de la patte (10, 11) de l'élément d'implant (1, 3, 4, 5) adjacent est formé par un bord de la perforation (9) de l'élément d'implant (2) adjacent.

5. Implant vertébral selon l'une des revendications 1 à 4, **caractérisé en ce qu'**il est prévu quatre éléments d'implant, à savoir les deux éléments extérieurs (1, 4), qui présentent respectivement un côté d'ancrage (6, 7) orienté vers la vertèbre adjacente, l'implant intercalé formé par un élément principal central comportant les deux bords d'appui (14, 15) et au moins un élément d'implant (3, 5) servant d'élément de raccord pour raccorder chacun des deux éléments d'implant extérieurs (1, 4) à l'élément principal central (2).

6. Implant vertébral selon l'une des revendications 2 à 5, **caractérisé en ce que** des perforations transversales (16, 17) communiquant avec les perforations axiales (9) sont associées aux éléments d'implant (2, 3, 5) pourvus de bords d'appui destinés à saisir les bords de retenue (12, 13) des pattes (10, 11) introduites, au travers desquelles perforations transversales les pattes (10, 11) destinées à assurer la connexion par encliquetage des éléments d'implant consécutifs peuvent être sollicitées en vue d'une déformation élastique latérale permettant la rupture de la connexion par encliquetage.

7. Implant vertébral selon l'une des revendications 1 à 6, **caractérisé en ce que** les éléments d'implant (1 à 5) sont de forme parallélépipédique et sont pourvus, au niveau d'un de leur bords, sur leur côté orienté vers l'élément d'implant adjacent en direction de l'élément principal (2), d'un évidement (18) dans lequel s'engage une partie en saillie (19) prévue sur l'élément d'implant contigu.

8. Implant vertébral selon la revendication 7, **caractérisé en ce que** l'évidement est formé par un chanfrein (18) coopérant avec une partie en saillie (19) prévue sur l'élément d'implant contigu sous la forme d'une moulure prismatique.
